# EUROPEAN PATENT APPLICATION

(11) **EP 1 775 589 A1**
(43) Date of publication of application: **18.04.2007**
(21) Application number: 05768495.3
(22) Date of filing: 04.08.2005
(51) Int. Cl.: G01N 33/53, C12M 1/00, C12M 1/40, C12Q 1/68, G01N 21/64, G01N 21/78, G01N 33/566, G01N 35/04, G01N 37/00

(54) **DNA CHIP MANUFACTURING METHOD, MANUFACTURING SYSTEM, HYBRIDIZATION DETECTION METHOD, DETECTION SYSTEM, SUBSTRATE TREATMENT DEVICE, AND SUBSTRATE TREATMENT METHOD**

(30) Priority: 05.08.2004 JP 2004229837
(71) Applicant: SONY CORPORATION, Tokyo 141-0001 (JP)
(72) Inventor: MAMINE, Takayoshi c/o Sony Corporation, Tokyo (JP); SAKAMOTO, Yasuhiro c/o Sony Corporation, Tokyo (JP)
(74) Representative: Robinson, Nigel Alexander Julian
(86) International application number: PCT/JP2005/014314
(87) International publication number: WO 2006/018981

(57) **Abstract**

Disclosed herein is a technology relating to DNA chips for efficient hybridization in a short time and accurate detection of hybridization. It employs a discoid substrate 1 or a DNA chip 10 provided with detecting elements 3, each having at least a reaction area R for hybridization and opposing electrodes (such as E₁ and E₂) which are so arranged as to apply an electric field to a medium held in said reaction area. The opposing electrodes apply an electric field which immobilizes the nucleic acid D for detection onto the detection surface U, brings about hybridization between the nucleic acid D for detection and the target nucleic acid T, and removes excess substances B. The effect of these actions is efficient hybridization and accurate detection of hybridization.

## Description

### [Technical Field]

The present invention relates to a technology of DNA chips and, more particularly, to a method and system for production of a DNA chip, a method and system for detection of hybridization, and an apparatus and method for treatment of a substrate.

### [Background Art]

The technique developed in 1970s for assay to detect hybridization mostly employed porous materials such as nitrocellulose membrane or the like, radioactive labeling, and autoradiography.

The recent innovation in this field is the so-called DNA chip or DNA microarray (which are collectively referred to as DNA chip hereinafter) for microassay. A DNA chip is an integrated substrate on which specific DNA molecules are minutely arranged by the microarray technology. It is used for analysis of gene mutation, analysis of SNPs (single nucleotide polymorphisms), and analysis of gene expression frequency. Now, it plays an important role in various fields including drug development, clinical diagnosis, pharmacogenomics, study of evolution, and legal medicine.

A DNA chip has a large variety and number of DNA oligochains and cDNA (complementary DNA) integrated on a glass substrate or silicon substrate. Therefore, it allows for comprehensive analysis of hybridization.

Analysis by a DNA chip is accomplished in the way briefly explained below. The procedure starts with extraction of mRNA from cells or tissues. The extracted mRNA is amplified by PCR, during which it is incorporated with fluorescence probe dNTP by reverse transcription PCR reaction. The amplified mRNA is hybridized with a DNA probe held on a glass substrate or silicon substrate. The resulting hybridization is detected by fluorescence measurement.

At present, DNA chips are produced by using several technologies including photolithography which uses semiconductor exposure technology, ink jetting which is based on piezoelectric technology, and mechanical microspotting. Although they have both merits and demerits, they need a non-porous glass or plastics substrate having a smooth, uniform surface which allows for analysis with a very small amount of sample without diffusing or absorbing samples.

DNA chips fall under two broad categories. The first category includes those which have oligonucleotides synthesized directly on a specific substrate by photolithography mentioned above. They are commercially available from Affimetrix. (See JP-A-Hei 4-505763.) DNA chips of this type have a high degree of integration but have only several tens of bases (only several tens) because of limitation of DNA synthesis on a substrate.

The second category includes those which are called "Stanford type". They have previously prepared DNA dispensed and fixed on a substrate by a split pin. (See JP-A-Hei 10-503841.) DNA chips of this type have a lower degree of integration than those mentioned above but have DNA fragments as long as 1 kb.

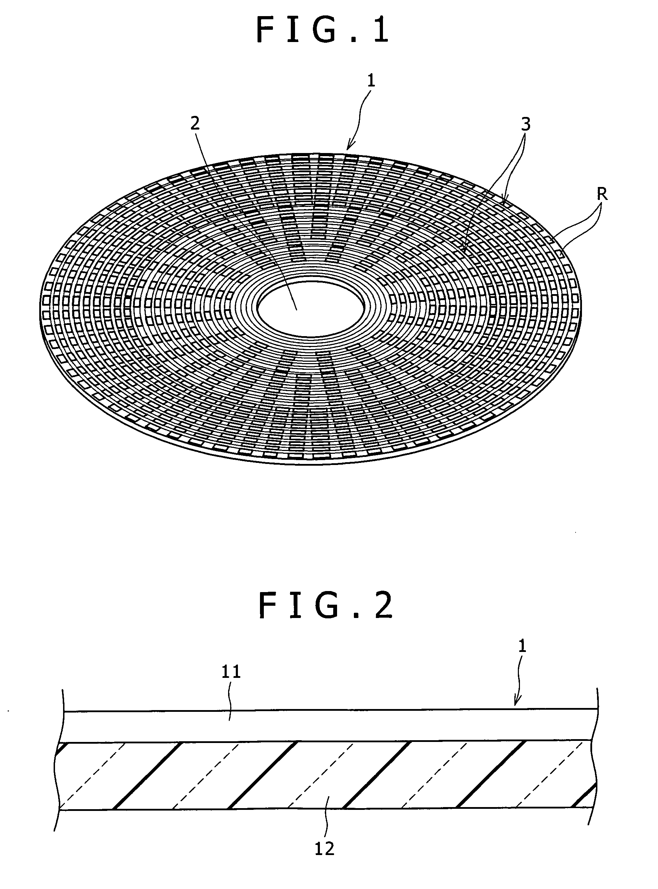

Currently available DNA chips mostly have divided sections on a rectangular substrate as disclosed in Japanese Patent Laid-open No. 2000-60554 (Claim 1 and Fig. 1). Each section has a probe for hybridization. Incidentally, this disclosure also mentions that each section may contain a gel through which a sample of polynucleotide migrates by electrophoresis.

It is common practice to employ a fluorescence scanning system to detect or read hybridization that has occurred in a specific reaction area on a DNA chip. This system is designed to detect fluorescence from a fluorescence substance that labels nucleic acid molecules.

The fluorescence detecting system irradiates the fluorescence substance with specific excitation light and detects fluorescence emitted by the fluorescence substance. It improves the speed and accuracy of detection and contributes to safety because it employs the fluorescence substance in place of conventional radioactive substances.

Detection of fluorescence is accomplished by using an imaging technique with a confocal scanner or a CCD camera. The detecting device converts fluorescence emanating from the DNA chip into digital outputs for subsequent determination of the data files and analysis.

It is known that an electric field acts on a charged substance. To be concrete, an electric field stretches or moves double-strand nucleic acid molecules in the liquid phase. This phenomenon is due to ion cloud resulting from negative charges (phosphate ions as skeletons) and positive charges (ionized hydrogen atoms from surrounding water). These negative and positive charges give rise to dipoles, which are aligned in one direction as a whole upon application of a high-frequency high voltage. As the results, those molecules involved are stretched and, in the presence of an uneven electric field, are moved to a position where electric flux lines concentrate. (See Seiichi Suzuki, Takeshi Yamanashi, Shin-ichi Tazawa, Osamu Kurosawa, and Masao Washizu; "Quantitative analysis on electrostatic orientation and DNA in stationary AC electric field using fluorescence anisotropy", IEEE Transaction on Industrial Applications, Vol. 34, No. 1, p. 75-83 (1998).)

It has been reported that DNA molecules existing in a randomly coiled form in a solution are stretched straight parallel to a high-frequency electric field (1 MV/m, 1 MHz) applied across minute electrodes having a gap of tens to hundreds of micrometers. The electric field brings about induced polarization and the polarized DNA molecules are attracted naturally to the electrode edge by the electrodynamic effect called "electrophoresis". Finally, the DNA molecules are immobilized, with their ends in contact with the electrode edge. (See Masao Washizu, "DNA handling under observation", Visualized Information, Vol. 20, No. 76 (January, 2000).)

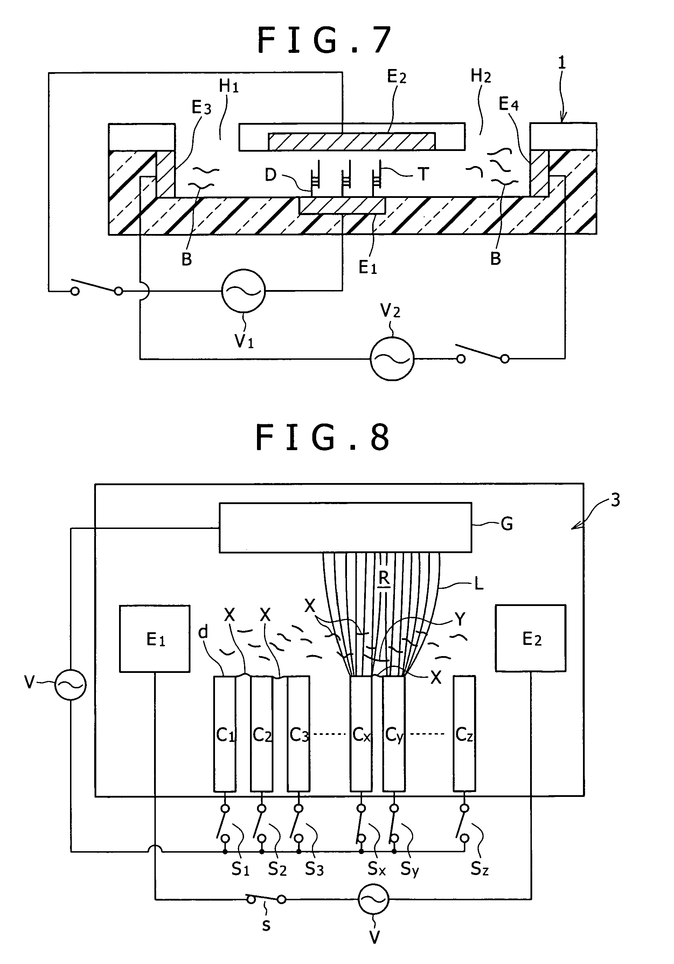

Another DNA chip technology that utilizes an electric effect is embodied in an apparatus for detection of biopolymers which is designed to separate DNA molecules according to whether they have formed complementary strands or to stretch DNA molecules by application of a DC voltage to the DNA probe. (See Japanese Patent Laid-open No. 2002-168864, Paragraph 0015 and Fig. 7.) Moreover, there has been proposed a DNA chip which has electrode pins and nucleotide molecules immobilized onto them. (See Japanese Patent Laid-open No. 2001-241315.)

Various DNA chip technologies proposed so far are designed to analyze hybridization between a nucleic acid such as a probe DNA for detection and its complementary target nucleic acid, the former being immobilized onto a reaction area previously formed in a substrate.

Conventional DNA chip technologies, however, are not advanced enough for their wide spread use because of their limited efficiency in hybridization. This drawback arises from steric hindrance and contaminants. Hybridization is subject to steric hindrance because nucleic acid molecules assume the rounded or twisted high-order structure (in randomly coiled shape), and steric hindrance prevents smooth complementary linkages at bases. Hybridization is also inhibited when the substrate has contaminants around the nucleic acid for detection. Solution to these problems will greatly reduce time required for assay with DNA chips.

In addition, conventional DNA chip technologies suffer the disadvantage of causing false positive and false negative due to unnegligibly frequent mishybridization. In other words, they need improvement in accuracy of detection.

Accurate detection needs adequate conditions and environment for accurate hybridization. It also needs the reaction area to be cleared of unnecessary substances that interfere with detection by a specific aqueous solution prior to detection. The clearing step must be carried out adequately without adverse effect on the accuracy of detection.

For the comprehensive analyses by hybridization to be used more widely in the future, it will be necessary to develop new technologies to cope with the increasing amount of information on the substrate, the simultaneous analyses of a large number and variety of genes, the simplified method of analysis, and the cost reduction of apparatus and chip substrates.

Thus, it is an object of the present invention to provide a method and system for production of a DNA chip, a method and system for detection of hybridization, and an apparatus and method for treatment of a substrate, which will contribute to efficient manipulation of gene information on the substrate and efficient hybridization in a short time and accurate detection of hybridization.

### [Disclosure of Invention]

The present invention covers (1) a method for production of a DNA chip, (2) a system for production of a DNA chip, (3) a method for detection of hybridization, (4) a system for detection of hybridization, (5) an apparatus for treatment of a substrate, and (6) a method for treatment of a substrate.

(1) Method for production of DNA chip

The first embodiment of the present invention is directed to "a method for production of a DNA chip" from a substrate having detecting elements, each provided with at least a reaction area for hybridization and opposing electrodes so arranged as to apply an electric field to a medium held in said reaction area.

The method includes a step of performing surface treatment on a substrate, a step of applying through the opposing electrodes an electric field to the medium containing a previously prepared nucleic acid for detection which is held in the reaction area, thereby immobilizing the nucleic acid for detection onto the electrode surface, and a step of removing an excess of the nucleic acid for detection from the reaction area.

The step of surface treatment of a substrate is intended to make the substrate compatible with the medium. The reaction area holds the medium which is dropped or injected thereinto.

The medium may be dropped or injected while the substrate is rotating. The step of application of an electric field includes an additional step of moving the nucleic acid for detection by electrophoresis induced by application of an electric field. The electric field is a high-frequency one having a voltage no lower than 1 MV/m and a frequency no lower than 1 MHz.

(2) System for production of a DNA chip

The second embodiment of the present invention is directed to a system for production of a DNA chip from a substrate having detecting elements, each provided with at least a reaction area for hybridization and opposing electrodes so arranged as to apply an electric field to a medium held in said reaction area.

The system includes means for dropping or injecting a medium containing a previously prepared nucleic acid for detection into the reaction area, and means for applying an electric field through the opposing electrodes to the medium held in the reaction area.

The system further includes means for dropping or injecting the medium into the reaction area and permitting the medium to be held in the reaction area and means for removing an excess of the nucleic acid for detection from the reaction area. The means for application of an electric field is one which applies a high-frequency electric field. The system further includes means for rotating the substrate. The means for dropping or injection supplies the medium to the reaction area works in synchronism with rotation of the substrate.

(3) Method for detection of hybridization

The third embodiment of the present invention is directed to a method for detection of hybridization on a substrate having detecting elements, each provided with at least a reaction area for hybridization, opposing electrodes so arranged as to apply an electric field to a medium held in the reaction area, and a nucleic acid for detection which is immobilized in the reaction area.

The method includes a step of dropping or injecting a medium containing a previously prepared nucleic acid for detection into the reaction area, a step of applying an electric field through the opposing electrodes to the medium held in the reaction area, a step of causing hybridization to proceed between the nucleic acid for detection and the target nucleic acid, a step of dropping or injecting an intercalator into the reaction area, and a step of irradiating the reaction area with excitation light of specific wavelength and detecting the intensity of the resultant fluorescence. Incidentally, the step of applying an electric field may be performed on the entire surface of the substrate at one time or on the divided regions of the substrate sequentially.

The step of "dropping or injecting a medium containing a target nucleic acid into the reaction area" and the step of "dropping or injecting an intercalator into the reaction area" may be carried out at the same time. Hybridization may be carried out in the presence or absence of electric field.

For improved accuracy of detection, the method may be carried out such that the step of detection of fluorescence is preceded by a step of removing an excess of the target nucleic acid existing in the reaction area from the hybridization area. This additional step of removing excess target nucleic acid may be accomplished by application of an electric field which attracts excess target nucleic acid to the surface of the electrode outside the hybridization area.

The method for detection of hybridization may be carried out such that the excitation light is directed to the lower side of the substrate. It may also be carried out such that the step of dropping or injecting the medium (containing the previously prepared target nucleic acid) into the reaction area, the step of dropping or injecting the intercalator into the reaction area, and the step of irradiating the reaction area with the excitation light of specific wavelength are performed while the substrate is rotating.

(4) System for detection of hybridization

The fourth embodiment of the present invention is directed to a system for detection of hybridization on a substrate having detecting elements, each provided with at least a reaction area for hybridization, opposing electrodes so arranged as to apply an electric field to a medium held in the reaction area, and a nucleic acid for detection which is immobilized in the reaction area.

The system includes at least means for dropping or injecting a medium containing a previously prepared target nucleic acid into said reaction area, means for applying an electric field through the opposing electrodes to the medium held in the reaction area, means for causing hybridization to proceed between the nucleic acid for detection and the target nucleic acid, means for dropping or injecting an intercalator into the reaction area, and means for irradiating the reaction area with excitation light of specific wavelength and detecting the intensity of the resultant fluorescence.

The system for detection of hybridization may be modified such that the substrate has a layer that transmits the excitation light and the excitation light is emitted from a source facing the reverse side of the substrate. This structure permits the arrangement of the means for dropping or injecting the medium above the substrate and the arrangement of the optical means for detection of hybridization under the substrate. The arrangement in this manner makes the entire apparatus compact and simplifies the structure of the apparatus.

The system may further include means for controlling temperature and/or means for controlling humidity for hybridization and means for supplying water to the reaction area.

The system may further include means for rotating the discoid substrate and a servo control means for positioning the detecting element on the substrate and a servo control means for focusing.

(5) Apparatus for treatment of substrate

The fifth embodiment of the present invention is directed to an apparatus for treating a substrate having thereon reaction areas for hybridization. The apparatus includes means for supplying a target nucleic acid to the reaction areas where a nucleic acid for detection has been immobilized, means for heating to a controlled temperature the substrate which has been supplied with the target nucleic acid, means for reading the state of hybridization on said substrate, and means for transporting the substrate. The apparatus may additionally have means for applying an electric field to the substrate or means for optical reading.

The apparatus may be modified such that the means for transportation continuously transports the substrate through the means for supplying, the means for heating, and the means for reading. The apparatus may be modified such that the means for supplying, the means for heating, and the means for reading are arranged in series. The apparatus may also be modified such that the means for supplying has means for humidifying.

The apparatus may further include means for introducing the substrate to the means for supplying and means for discharging the substrate from the means for reading. The means for introducing the substrate and the means for discharging the substrate can be combined into one unit. The apparatus may have the means for supplying, the means for heating, the means for reading, the inlet for the substrate, and the outlet for the substrate arranged in series.

The apparatus may additionally have a second means for supplying the solvent to the reaction area in which the nucleic acid for detection is immobilized, or means for extracting nucleic acids from blood.

(6) Method for treatment of substrate

The sixth embodiment of the present invention is directed to a method for treating a substrate having thereon reaction areas for hybridization. This method includes a step of supplying a target nucleic acid to the reaction areas where a nucleic acid for detection has been immobilized, a step of heating to a controlled temperature the substrate which has been supplied with the target nucleic acid, and a step of reading the state of hybridization in the reaction area, with the steps being performed consecutively.

The method may have an additional step of applying an electric field to the substrate. Application of an electric field in the step of supplying nucleic acids or during hybridization gives an electrodynamic effect to the nucleic acid molecules, thereby improving the efficiency of hybridization and the accuracy of detection of hybridization.

The method may be modified such that the step for temperature control gives a period in which hybridization takes place between the nucleic acid for detection and the target nucleic acid.

The method may be modified such that reading is accomplished by optical reading, the substrate is transported through the step of supplying the nucleic acid, the step of controlling temperature, and the step of reading. The method may be modified such that the step of supplying said nucleic acid involves humidification.

The following is the definition of the technical terms used in the present invention.

"Nucleic acid for detection" is a nucleic acid which is free or stationary in a medium held in the reaction area and which functions as a probe to detect a nucleic acid having a complementary base sequence for specific reaction with said nucleic acid for detection. Its typical example is oligonucleotide or polynucleotide as a DNA probe. "Target nucleic acid" is a nucleic acid which has a base sequence complementary to said nucleic acid for detection.

"Nucleic acid" is a polymer (or nucleotide chain) of phosphate ester of nucleoside in which a purine or pyrimidine base and a sugar are combined together through the glycoside linkage. It embraces DNA (complete or fragmentary), including probe DNA, which is formed by polymerization from oligonucleotide, polynucleotide, purinenucleotide, or pyrimidinenucleotide. It also embraces cDNA (c probe DNA) formed by reverse transcription, RNA, and polyamide nucleotide derivative (PNA).

"Hybridization" is a reaction to form complementary strands (double strands) having a complementary base sequence. "Mishybridization" is a reaction to form abnormal complementary strands. It may be called "Mishybrid" in this specification.

"Reaction area" is an area in which hybridization and other reactions take place. It includes a well-shaped one which holds a liquid or gel. Reactions in the reaction area are not specifically restricted so long as they accord with the object and effect of the present invention.

"Opposing electrodes" denotes at least a pair of electrodes whose surfaces face each other. "Axis of opposition" is a straight line connecting the centers of the surfaces of the opposing electrodes. "Crossing" includes two-dimensional ones with a point of intersection and three-dimensional ones without a point of intersection. The angle of crossing is not specifically restricted so long as it accords with the object and effect of the present invention.

"Excess substances" includes an excess of the nucleic acid for detection such as probe DNA or the like present in the reaction area, an excess of the target nucleic acid having a base sequence complementary to the nucleic acid for detection, and an excess of "intercalator" to be inserted into the complementary strands obtained by hybridization.

"Intercalator" is a substance labeled with fluorescence which is inserted into the double-strand nucleic acid. It is used for detection of hybridization. It includes POPO-1 and TOTO-3.

"Steric hindrance" means a phenomenon that a desired reaction (or hybridization in this invention) is hindered by bulky groups in molecules or by arrangement or three-dimensional structure (high-order structure) of molecules which prevent reacting groups from coming close each other.

"Electrophoresis" is a phenomenon that molecules migrate in the presence of an uneven electric field. It occurs when either a DC voltage or an AC voltage is applied. In the latter case, the reversing voltage polarity reverses the direction of polarization of molecules. (See "Micromachine and Material technology" from CMC, edited by Teru Hayashi, p. 37-46, Chapter 5, Manipulation of Cells and DNA.)

"DNA chip" denotes a substrate for detection of hybridization on which nucleic acid molecules such as DNA probe or the like are immobilized at very small intervals. It also includes DNA microarray.

The present invention allows for efficient integration (immobilization) of gene information on a substrate and efficient hybridization and its accurate detection in a short time.

### [Brief Description of Drawings]

[Fig. 1] Fig. 1 is a perspective view showing a typical example of a discoid substrate suitable for use in the present invention.
[Fig. 2] Fig.2 is a partly sectional view showing the basic layered structure of the substrate 1.
[Fig. 3] Fig. 3 is a schematic enlarged view of the detecting surface U formed in the reaction area R.
[Fig. 4] Fig. 4 is a sectional view of the detecting element 3 with a pair of opposing electrodes arranged therein in one way.
[Fig. 5] Fig. 5 is a sectional view of the detecting element 3 with a pair of opposing electrodes arranged therein in another way.
[Fig. 6] Fig. 6 is a sectional view of the detecting element 3 with a pair of opposing electrodes arranged therein in further another way.
[Fig. 7] Fig. 7 is a sectional view of the detecting element 3 with two pairs of opposing electrodes arranged therein such that their axes of opposition orthogonally intersect each other.
[Fig. 8] Fig. 8 is a schematic diagram showing the arrangement of scanning electrodes C in the detecting element 3.
[Fig. 9] Fig. 9 is a schematic diagram showing the patterning of the detecting surface U.
[Fig. 10] Fig. 10 is a schematic diagram showing one embodiment of the detecting element 3 which are provided with electrodes having projections.
[Fig. 11] Fig. 11 is a schematic diagram showing one embodiment of the wiring 7 for electric power supply to the opposing electrodes.
[Fig. 12] Fig. 12 is a flow sheet showing the steps for production of the DNA chip 10.
[Fig. 13] Fig. 13 is another flow sheet showing the steps for production of the DNA chip 10.
[Fig. 14] Fig. 14 is a block diagram showing an embodiment of the system pertaining to the present invention.
[Fig. 15] Fig. 15 is a schematic diagram showing an embodiment of the apparatus for treatment of the substrate, the apparatus being a combination of the constituents of the system shown in Fig. 14.
[Fig. 16] Fig. 16 is a schematic block diagram showing the system for treatment of the substrate that can be used for production of the DNA chip and/or detection of hybridization.
[Fig. 17] Fig. 17 is a flow sheet showing one way of detecting hybridization.
[Fig. 18] Fig. 18 is a flow sheet showing another way of detecting hybridization.
[Fig. 19] Fig. 19 is a diagram showing the waveform used to apply a high-frequency electric field W₁ and subsequently a low-frequency electric field W₂.
[Fig. 20] Fig. 20 is a diagram showing the waveform used to apply a low-frequency electric field W₂ and subsequently a low-frequency electric field W₃ with a rectangular waveform.
[Fig. 21] Fig. 21 is a diagram showing the waveform used to apply a low-frequency electric field W₂ and subsequently a low-frequency electric field W₃, with a period of null electric field interposed between them.
[Fig. 22] Fig. 22 is a diagram showing the waveform used to apply a high-frequency electric field W₁ and subsequently a low-frequency electric field W₃, with a period of DC electric field W₄ interposed between them.
[Fig. 23] Fig. 23 is a diagram showing the waveform used to apply a high-frequency electric field W₁ and subsequently a low-frequency electric field W_{3,} with a period of DC electric field W₄ placed before W₁.
[Fig. 24] Fig. 24 is a diagram showing the waveform used to apply a high-frequency electric field W₅ with a superposed DC component and subsequently a low-frequency electric field W₂.
[Fig. 25] Fig. 25 is a diagram showing the waveform used to apply a high-frequency electric field W₁ and subsequently a low-frequency electric field W₆ with a superposed DC component.
[Fig. 26] Fig. 26 is a diagram showing the waveform used to apply a high-frequency electric field W₅ with a superposed DC component and subsequently a low-frequency electric field W₆ with a superposed DC component.

### [Best Mode for Carrying out the Invention]

The best mode for carrying out the invention will be described below with reference to the accompanying drawings. The illustrated embodiments are typical ones which are not intended to restrict the scope of the invention.

### <1. "Substrate" suitable for the embodiments of the present invention>

### (1) Basic structure of substrate

The present invention may employ any substrates made of the same material as used for optical information recording media such as CD (Compact Disc) and DVD (Digital Versatile Disc). The substrates pertaining to the present invention are not specifically restricted in shape; however, discoid ones are particularly desirable. The following description is concerned mainly with discoid substrates.

Fig. 1 shows an example of the discoid substrate suitable for use in the present invention. The illustrated discoid substrate 1 ("substrate 1" for short hereinafter) is formed from optically transparent quartz glass or plastics such as silicone, polycarbonate, and polystyrene or the like, particularly those plastics capable of injection molding. The substrate 1 formed from inexpensive plastics contributes to lower running costs than conventional glass chips.

The substrate 1 may have a hole 2 (not necessarily a through hole) of prescribed size at its center. This hole 2 permits the substrate to be held and rotated by a spindle 9 or chuck mentioned later inserted therein.

Also, the hole 2 may be so constructed as to receive an appropriate jig (not shown) which supplies electricity to all or part of the opposing electrodes (mentioned later) which are arranged on the substrate 1 to apply electric fields.

Fig. 2 is a partly sectional view showing the basic layered structure of the substrate 1. The substrate 1 includes essentially of a reflecting layer 11 and a light transmitting layer 12 for specific wavelengths. It should preferably be constructed such that the reflecting layer 11 is placed on the light transmitting layer 12 as shown in Fig. 2. This structure facilitates detecting operations etc. with the help of upward illumination through the reverse of the substrate 1.

The substrate 1 constructed as mentioned above may be incorporated into an entire system for experiment in such a way that those mechanical devices for dropping or injecting sample solutions are arranged above it and those optical devices for detection (or reading) are arranged below it.

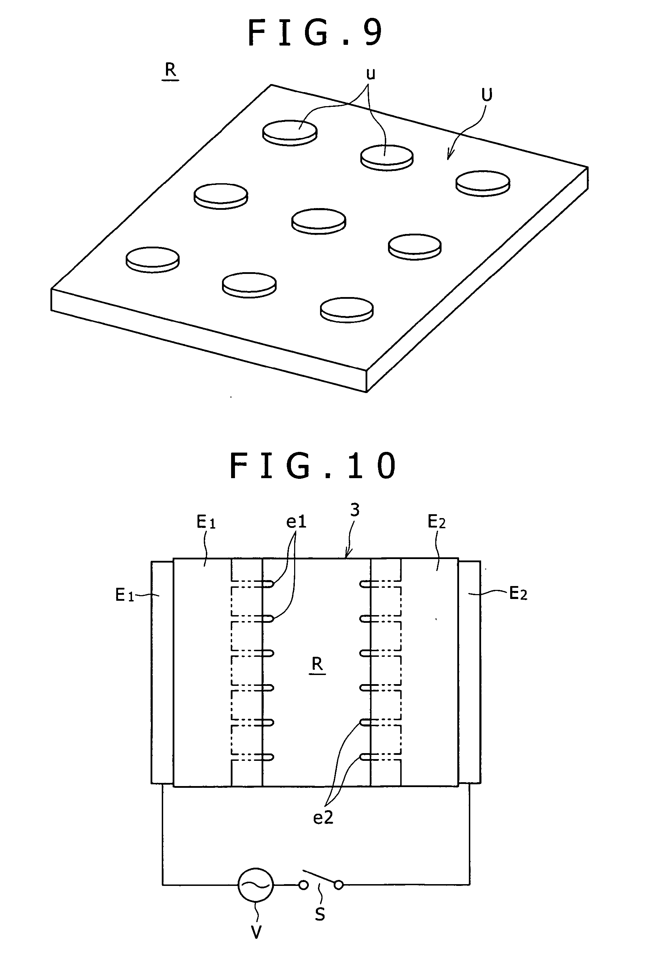

The substrate 1 is formed such that its reflecting layer 11 has a thickness of several to tens of nanometers. The reflecting layer 11 may be a single layer of metallic material or inorganic material which has a reflectance of 5% to 50% for laser beams from the servo mechanism (indicated by symbol V in Fig. 10).

The above-mentioned range of reflectance is high enough for stable servo mechanism (by focusing and tracking) and yet low enough for transmission of excitation light and fluorescence necessary for measurement of fluorescence intensity.

With the range of reflectance mentioned above, the substrate 1 eliminates completely disturbance from the reflected laser beams for the servo mechanism (indicated by symbol V in Fig. 10) even when its refractive index is very close to that of any substance existing thereon.

The reflecting layer 11 may be a reflecting film in multilayer structure with wavelength selectivity which is formed from more than one inorganic material. Such a reflecting film reflects only the laser beams for the servo mechanism (indicated by symbol V in Fig. 10) without loss of excitation light and fluorescence for measurement of fluorescence intensity.

The reflecting film mentioned above functions as the reflecting layer 11 which has a laser beam reflectance higher than 50% (for example, 90% or above) without any adverse effect on measurement of fluorescence intensity.

The substrate 1 has a large number of detecting elements 3 formed in the light transmitting layer 12 or a reaction area forming layer (not shown) which is a layer of photosensitive polyimide resin or the like placed on the light transmitting layer 12. Each detecting element 3 includes a reaction area R resembling a well and a pair of opposing electrodes (or at least one electrode), which face each other. (Electrodes are too small to show in Fig. 1.) Each reaction area is where hybridization takes place between a DNA probe (nucleic acid for detection) and a target nucleic acid. The reaction areas can be formed by any known optical disc mastering technology. See Fig. 1.

The light transmitting layer 12 may be of double-layer structure (not shown), with the upper layer having a higher refractive index than that of the lower layer and medium. This structure allows for quick and accurate focusing and positioning servo control.

Each detecting element 3 has a detecting area (corresponding to a portion of the reaction area R) and a servo area. The servo area has a servo mark for radial position control and an address mark for address information of each detecting element 3 on the substrate 1.

The detecting elements 3 may be arranged in a circumferential or spiral pattern on the substrate 1 such that the detecting area and servo area appear alternately. The periodically arranged servo areas constitute the sample servo system known in the field of optical disc technology.

The servo marks and address marks can be formed by the conventional optical disc mastering process. If the substrate 1 is analogous to an optical disc, then the reaction areas R for sample dropping and detection are analogous to the user data areas. Other areas may have synchronous pits for tracking according to the sample servo system. The addresses that immediately follow them give the position information on the substrate 1.

The address part starts with a sector mark as a leading pattern. It includes a VFO (Variable Frequency Oscillator) showing the rotating phase of the substrate 1, an address mark showing the starting position of address data, and an ID (Identifier) holding the track and sector numbers.

Any position on the substrate 1 in its radial direction may be defined by the track position information obtained from a fine error signal and an address mark. The former is a difference in signal levels from two tracking marks which are shifted by a quarter of track toward the plus and minus sides in the radial direction.

The track position information allows for the positioning of the discharge head for medium supply and the optical head for detection while the substrate 1 is rotating on the radial stage.

Such positioning is controlled by the address-tracking error signals and focus error signals which are received by the optical pick-up from the reading side of the substrate 1 rotating on the radial stage.

The substrate 1 used in the present invention has its surface previously surface-treated for medium affinity. In other words, the substrate surface is divided into hydrophilic sections and hydrophobic sections. This provision saves sample aqueous solutions and smoothly leads organisms to the detecting areas.

(2) Structure of "detecting element 3"

The substrate 1 has a large number of small areas arranged thereon, which function as detecting elements. Each detecting element 3 has a reaction area R (or a well-like small area) which holds a sample-containing aqueous solution and a gel (such as agarose gel) so that hybridization takes place therein. Each reaction area R may have means to supply it with water to prevent medium evaporation from it.

These detecting elements 3 should be arranged on the substrate 1 in such a way that they can be easily grouped according to the object of assay. In other words, they may be regularly arranged at certain intervals in the circumferential, radial, or spiral direction on the discoid substrate 1. Moreover, they may be grouped by angles in the circumferential direction according to the kind and gene of samples.

The reaction area R in the detecting element 3 is not specifically restricted in shape and size; its length, width, and depth usually ranges from several to hundreds of micrometers. They may be properly established according to the spot diameter of the excitation light and the minimum drop size of the sample solution (containing the nucleic acid for detection and the target nucleic acid).

(3) Structure of "detecting surface U"

The reaction area R in the detecting element 3 has a treated surface for immobilization of DNA probe which is nucleic acid for detection. This treated surface is referred to as "detecting surface" hereinafter.

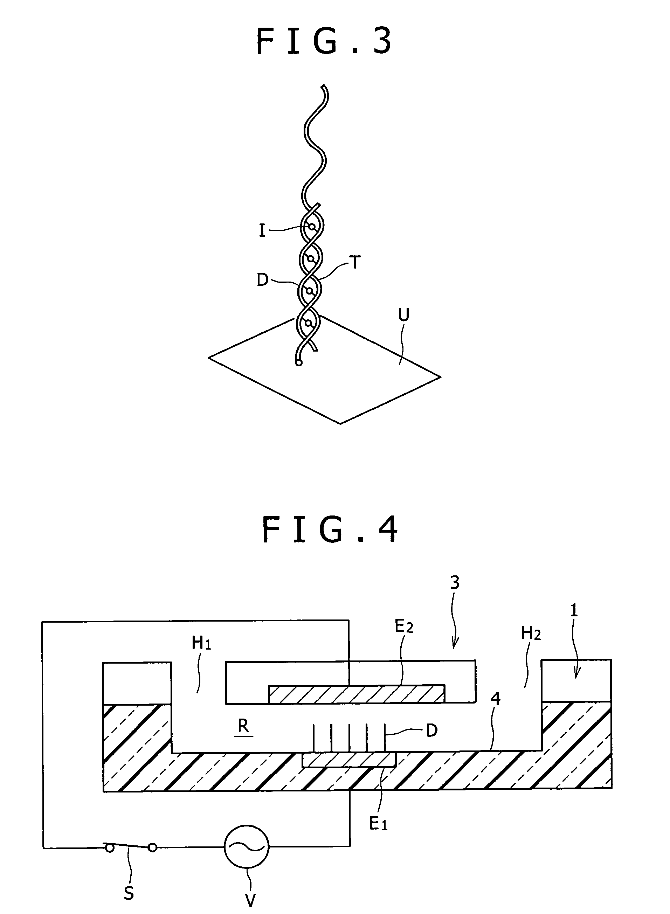

Fig. 3 is a schematic enlarged view of the detecting surface U formed in the reaction area R. Fig. 3 schematically shows the detecting surface U, the nucleic acid D for detection immobilized thereon, the target nucleic acid T hybridized therewith, and the intercalator I connecting the double strands with each other.

The detecting surface U may be the surface of metal, such as gold, thin film or the surface of the electrode facing the reaction area R.

The detecting surface U (or the electrode surface) may be previously treated with a solution of amino-group containing silane coupling agent or a solution of lysine, for easy immobilization of DNA probe or the nucleic acid D for detection thereon.

The above-mentioned surface treatment on a plastics substrate may be preceded by plasma treatment and irradiation with deep UV light or far-infrared rays followed by treatment with a solution of amino-group containing silane coupling agent.

Alternatively, the plastics substrate may be coated by sputtering with a thin film of copper, silver, aluminum, or gold, which is subsequently coated with a substance having functional groups such as amino, thiol, and carboxyl groups or with cysteamine or streptavidine.

Surface treatment with streptavidine is suitable for the detecting surface that immobilizes the terminal of biotinylated DNA probe. Likewise, surface treatment with thiol (SH) groups is suitable for the detecting surface that immobilizes the nucleic acid D for detection which may be a probe DNA with a thiol-modified terminal through the disulfide (-S-S-) linkage.

The detecting surface U may optionally have inserting molecules connected thereto, such as linkers and spacers which help immobilize the nucleic acid D for detection. Such molecules provide a certain distance between the detecting surface U and the nucleic acid D for detection to prevent their mutual interference. In this way it is possible to prevent the nucleic acid D for detection from sticking to the detecting surface U except for its specific part to be immobilized.

In addition, the detecting surface U may also have inserting molecules (linkers) differing in molecular length connected alternately thereto at certain intervals, so as to prevent mutual interference between the molecules of the nucleic acid D for detection which have been immobilized onto the detecting surface U.

The inserting molecules for the above-mentioned object may have an adequate molecular length according to the length (the number of bases) of the nucleic acid D for detection or the target nucleic acid T or the distance of the molecules of the nucleic acid D for detection. In the case of hybridization between the nucleic acid D for detection which is a stretched probe DNA having 50 to 100 bases and the target nucleic acid T having about 1,600 bases, the moiety of extra base sequence (or the moiety of sequence which does not form the complementary strand) forms a randomly coiled mass of molecules due to Brownian motion. Such a mass has a diameter of 10 to 40 nm.

For the effective prevention of steric hindrance by the molecular mass of the target nucleic acid T, the inserting molecule (in its stretched state) should have a molecular length of 10 to 40 nm. In addition, the inserting molecules on the detecting surface U should be arranged at intervals of about 10 to 40 nm.

(4) Structure of "opposing electrodes"

The reaction area R of the detecting element 3 has at least a pair of opposing electrodes E₁ and E₂ which face the reaction area R as shown in Fig. 4. These opposing electrodes E₁ and E₂ may be formed from a metal, such as gold and aluminum, or a transparent conductor, such as ITO (indium-tin oxide). They are arranged such that they hold between them the detecting surface U in the reaction area R.

Fig. 4 shows how the paired opposing electrodes E₁ and E₂ are arranged to apply an electric field to the aqueous solution or medium, such as gel, held in the reaction area R. The opposing electrodes may be constructed when necessary from one electrode on the substrate 1 and the other electrode (E₂ shown in Fig. 4) which is an external electrode unit.

The opposing electrodes E₁ and E₂ apply an electric field, such as high-frequency alternating electric field (mentioned later) to produce the electrodynamic effect or electrophoresis, which stretches the nucleic acid molecules (nucleic acid D for detection and target nucleic acid T) in the medium, moves them toward the electrode edge where the electric flux lines concentrate, moves them while stretching them, or attracts them. Incidentally, the symbol D in Fig. 4 schematically shows the nucleic acid for detection which is immobilized in its stretched form. The opposing electrodes E₁ and E₂ may be used for electrophoresis or electroosmosis as desired.

Hybridization usually takes place between two single-stranded molecules which are twisted or randomly coiled into a ball. Therefore, it is subject to steric hindrance, which leads to inefficient complementary reaction and mishybridization.

The foregoing is avoided by application of an electric field through the opposing electrodes E₁ and E₂, which stretches nucleic acid molecules in high-order structure or moves them to an area where there is a high probability for association of nucleic acid molecules.

Hybridization in the presence of electric field proceeds extremely efficiently and accurately on account of reduced steric hindrance. This leads to a high-speed detection by hybridization. Incidentally, hybridization may be made to proceed by natural Brownian motion in the absence of electric field.

As shown in Fig. 4, the opposing electrodes in the reaction area R are connected to the external power source V through the switch S which turns on and off the electric field.

In addition, they have a control means for adjustment of field intensity, switching between AC and DC, and switching between high-frequency electric field or low-frequency electric field.

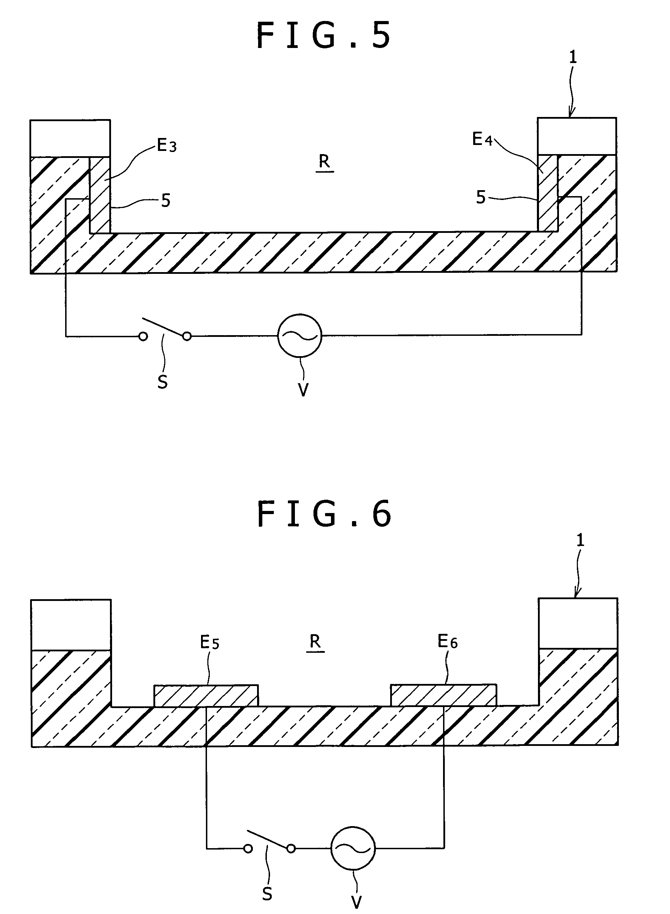

The opposing electrodes in the reaction area R may be arranged in any of the following three ways.
(1) One electrode E₁ is placed on the bottom 4 of the reaction area R and the other electrode E₂ is placed above it, as shown in Fig. 4.
(2) Two electrodes E₃ and E₄ are placed on the opposing vertical walls 5 of the reaction area R, as shown in Fig. 5.
(3) Two electrodes E₅ and E₆ are placed side by side (with a certain distance apart) on the bottom 4 of the reaction area R, as shown in Fig. 6.

Alternatively, more than one pair of opposing electrodes may be used. For example, two pairs of opposing electrodes may be arranged in the reaction area R such that their axes of opposition orthogonally intersect each other. As shown in Fig. 7, the reaction area R may have a first pair of opposing electrodes E₁ and E₂, which are vertically arranged, and a second pair of opposing electrode E₃ and E₄, which are horizontally arranged.

In the foregoing case, the first pair of opposing electrodes E₁ and E₂ apply a high-frequency electric field to adjust the high-order structure of the nucleic acid D for detection and to improve the efficiency of hybridization, and the second pair of opposing electrodes E₃ and E₄ apply an electric field to remove unnecessary substances (such as excess nucleic acid D for detection, excess target nucleic acid T, and excess intercalator I) which impede the detection of hybridization, and to move the hybridized nucleic acid molecules from the detection surface to another area. The use of an electric field for cleaning is a new technology that will replace the conventional cleaning process with an aqueous solution.

The above-mentioned cleaning operation by an electric field may be improved by combination with a low-frequency electric field. In other words, a low-frequency electric field applied to the reaction area R gives adequate vibration to the molecules of mishybridized nucleic acids, thereby dissociating them from the nucleic acid D for detection. The low-frequency electric field used for this purpose should have an intensity low enough not to dissociate the molecules of normally hybridized nucleic acids.

The reaction area R of the detecting element 3 may have a provision to supply the medium containing a sample for assay by capillary action. This provision may includes an inlet H₁ communicating with the reaction area R and a vent hole H₂ open to outside, as shown in Figs. 4 and 7. In addition, the inlet H₁ may have an opening whose surrounding surface is hydrophobic (opposite affinity for the medium).

The reaction area R of the detecting element 3 may additionally have scanning electrodes C arranged therein, as shown in Fig. 8. Fig. 8 shows the arrangement of scanning electrodes in the detecting element 3 briefly. The scanning electrodes C are placed between the opposing electrodes E₁ and E₂ such that their edges d face the common electrode G.

The embodiment shown in Fig. 8 has a series of scanning electrodes (C₁, C₂ ... C_{z}) connected respectively to a series of switches (S₁, S₂ ... S_{z}). The switches are turned on and off sequentially at certain time intervals to apply an electric field sequentially to the adjacent scanning electrodes (say, C₁-C₂, C₂-C₃, ...). The electric field fixes the molecule of the nucleic acid for detection (indicated by the symbol X) stretching between the edges d and d of the adjacent scanning electrodes. Incidentally, the symbol L in Fig. 8 schematically shows the electric flux lines that concentrate at the edges d of the scanning electrodes (Cₓ and C_{y}).

As mentioned above, the opposing electrodes facing the reaction area R may have one of their surfaces function as the detecting surface U (shown in Fig. 3) that immobilizes a DNA probe or a nucleic acid D for detection.

In this case, the electrode surface functioning as the detecting surface U should preferably be smaller than the other electrode surface. As shown in Figs. 4 and 7, the electrode E₁ which has a smaller surface area than its counter electrode concentrates electric flux lines to produce an uneven electric field, which causes nucleic acid molecules to readily migrate to the electrode E₁ by electrophoresis.

The electrode surface or the detecting surface U for immobilization may be irregularly roughened or regularly patterned with islands, as shown in Fig. 9 so that the projections thereof concentrate electric flux lines, thereby producing uneven electric fields. The regular pattern may be formed with convex or concave cones or with U-shaped holes which are not shown.

The roughening of the electrode surface may be accomplished by any known method such as sputtering deposition, epitaxy deposition, and etching. The roughening method is not specifically restricted.

The electrode surface should preferably be coated with an insulating material such as SiO₂, SiN, SiOC, SiC, SiOF, and TiO₂. The resultant insulating layer avoids electrochemical reactions caused by ionic solutions remaining in the reaction area.

Incidentally, the opposing electrodes E₁ and E₂, which are so arranged as to apply an electric field to the medium held in the reaction area R of the detecting element 3, may have projections towards the reaction area R, as shown in Fig. 10.

The thus constructed opposing electrodes cause the tips e₁ and e₂ of the projections to concentrate electric flux lines, thereby readily producing an uneven electric field. Therefore, they properly function to immobilize the nucleic acid D for detection.

(5) Means for power supply to "opposing electrodes"

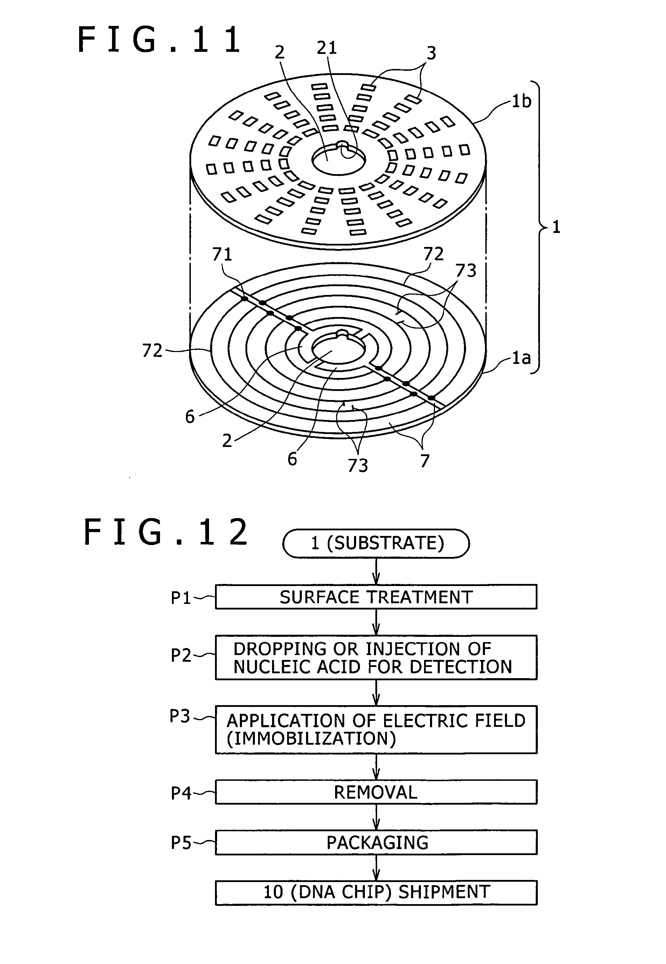

The opposing electrodes installed in the reaction area R may be supplied with electric power in any way without specific restrictions. According to one embodiment shown in Fig. 11, the substrate 1 is provided around its hole 2 with conducting parts 6 which come into contact with an external conducting jig (not shown) and also with power supply wirings 7 which extend from the conducting parts 6 to cover the entire surface of the substrate.

The conducting part 6 may be in the form of circle, ring, divided ring (shown in Fig. 11), etc. Incidentally, the symbol 21 in Fig. 11 denotes a notch for the positioning of the conducting jig or chuck.

The object of positioning may be achieved by forming a projection or making the hole 2 in a specific shape instead of employing the notch 21. The positioning method is not specifically restricted.

As shown in Fig. 11, one embodiment of the power supply wirings 7 includes the first to third wirings. The first wiring (main wiring) 71 radially extends from the conducting part 6 of the substrate 1. The second wiring 72 branches out in the circumferentially from the first wiring 71. The third wiring 73 branches out from the second wiring 72 and reaches the electrode. The second wiring 72 extends along concentric circles or spiral curves.

These wirings 71 to 73 may be formed in one or more layers on the wiring substrate 1a, which is accurately bonded to the detecting substrate 1b having the detecting elements 3 arranged thereon. Thus, the substrate 1 includes the two layers 1a and 1b.

The wiring 7 (wirings 71, 72, and 73) for power supply which are constructed as mentioned above can be used as the reference for revolution synchronizing signals or tracking signals for information reading. Thus they obviate the necessity of attaching signals and marks such as pits and bar codes to the substrate 1. This simplifies the structure of the substrate 1.

### <2. About the method for preparation of DNA chip>

The DNA chip according to the present invention is prepared from the discoid substrate 1, which is surface-treated and constructed as mentioned above.

As mentioned above, the substrate 1 has a large number of detecting elements 3 formed thereon, each includes at least a reaction area R in which hybridization takes place and a pair of opposing electrodes (say, E₁ and E₂) which apply an electric field to the medium held in the reaction area R. See Fig. 1. This substrate 1 functions as a base for the so-called DNA chip on which are immobilized specific nucleic acids D for detection.

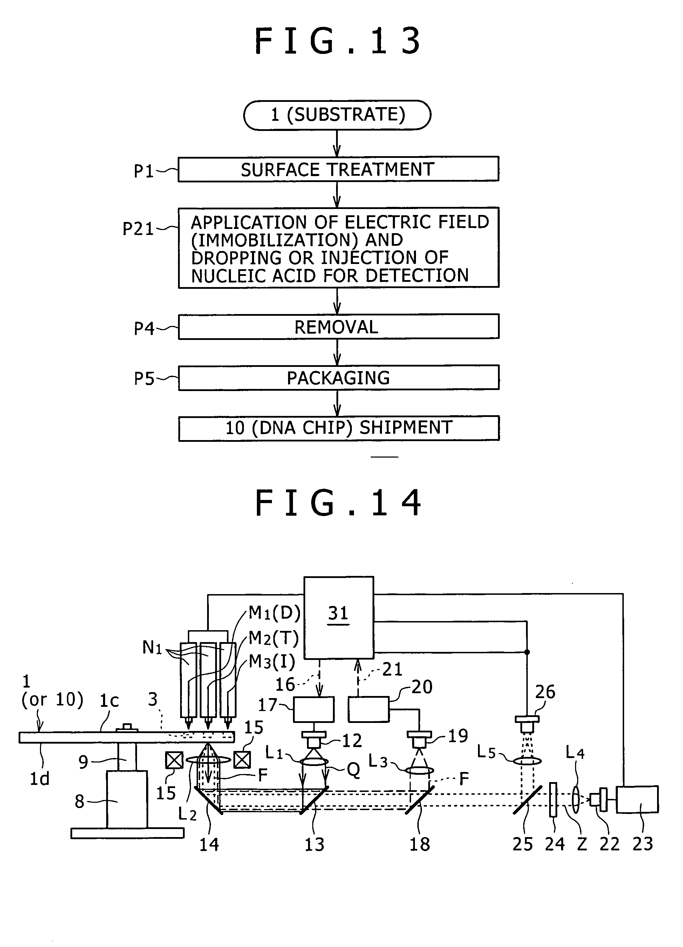

The DNA chip is prepared as follows by the process including the steps shown in Figs. 12 and 13 (flow sheets).

The entire process includes five steps. The first step P₁ is surface treatment on the substrate 1. The second step P₂ is dropping or injection of a medium containing a previously prepared nucleic acid D for detection into the reaction area R. The third step P₃ is application of an electric field to the medium held in the reaction area R by the opposing electrodes (say, E₁ and E₂).

The electric field applied in the third step P₃ produces the electrodynamic effect or electrophoresis which stretches the nucleic acid D for detection and immobilizes it onto the surface of the electrode (say, E₁) functioning as the detecting surface U. The fourth step P₄ is removal of an excess of the nucleic acid D for detection from the reaction area R. The fifth step P₅ is packaging and shipment to users. The foregoing steps will be described sequentially.

(1) The first step P₁ for surface treatment of the substrate

Surface treatment is performed as follows on the substrate 1 which has the reaction area R for hybridization. First, the substrate surface is treated with a hydrophobic substance, so that it is rendered hydrophobic. Second, the hydrophobic substrate surface is partly rendered hydrophilic.

The two steps of surface treatment may be properly combined to produce hydrophobic areas and hydrophilic areas on the substrate 1 as desired. For example, they may be combined such that the walls constituting the reaction area R are rendered hydrophilic and the other parts (non-reaction area) of the substrate 1 are rendered hydrophobic.

The reaction area R of the substrate 1 usually receives by dropping or injection an aqueous solution containing biomaterials. Biomaterials such as nucleic acids used for bioassay such as gene analysis are mostly hydrophilic.

Thus, the surface treatment is performed such that the reaction area R on the substrate 1 is rendered hydrophilic and the non-reaction area on the substrate 1 is rendered hydrophobic. The effect of the surface treatment in this manner is that sample solutions containing biomaterials are surely introduced into or caused to stay in the hydrophilic area of the reaction area R when they are dropped onto the substrate 1.

The above-mentioned step P₁ may be performed such that it renders only specific parts in the reaction area R on the substrate 1 hydrophilic or hydrophobic according to the object. For example, it may be performed such that it renders only the detection surface (or the electrode surface) in the reaction area R hydrophilic and only the other parts (or the non-detecting surface) in the reaction area R hydrophilic.

The thus formed hydrophilic and hydrophobic areas in the reaction area R permit hydrophilic or hydrophobic sample solutions and gels to be held in the desired positions or permit the nucleic acid for detection to be introduced into the desired position in the reaction area R for immobilization.

The hydrophobic surface may be formed by treating the substrate surface with an alkylsilane which is a hydrophobic substance represented by R-Si-X₁, X₂, X₃, where R is an alkyl group (which may contain an unsaturated group) and Xₙ denotes Cl or OR.

The step of partly rendering the hydrophobic surface hydrophilic may be accomplished by the reaction that forms hydroxyl groups on the hydrophobic surface. One way to introduce hydroxyl groups into an alkylsilane constituting the hydrophobic surface is by irradiation with UV light in the presence of oxygen which adds hydroxyl groups to the alkyl group of the alkylsilane.

(2) The second step P₂ for dropping or injection of the nucleic acid for detection

This step is to add, by dropping or injection, the previously prepared nucleic acid D for detection to the reaction area R on the substrate 1 which has undergone surface treatment as mentioned above. The nucleic acid D for detection may be a DNA probe such as DNA oligochain and cDNA (complementary DNA), which includes polynucleotides such as EST (expression sequence tag) and OFR (open reading frame) as cDNA fragments.

Dropping or injection may be accomplished in any way without specific restrictions. One way is by the ink jet printing technology which injects the sample solution containing a DNA probe to the reaction area R on the substrate 1 through a minute jet nozzle whose position is accurately controlled by a piezoelectric device utilizing the XYZ operation controlling system.

Another way is by the microspotting technology which drops the previously prepared nucleic acid for detection to the reaction area R on the substrate 1 through a microspotting pen, capillary, or tweezers attached to a print head under accurate position control by the XYZ operation controlling system.

The spotting technology makes it possible to add minute drops containing the nucleic acid D for detection and minute drops containing the target nucleic acid T accurately to the detecting element 3 on the substrate 1.

Incidentally, the ink jet printing technology uses the same nozzle as installed in an ink jet printer to electrically eject the nucleic acid for detection toward the substrate.

It includes the piezo ink jet method, the bubble jet method (registered trademark), and the ultrasonic jet method. The first method is designed to eject liquid drops by pressure produced by a piezoelectric element upon application of pulses. The second method is designed to eject liquid drops by pressure produced by bubbles which occur when the liquid is heated by a heater in the nozzle. The heater, which is embedded in a silicon substrate, is activated at regular intervals and controlled about at 300 °C /second to generate uniform bubbles for liquid ejection. This method, however, is not suitable for samples of biomaterials because the liquid is heated to a high temperature. The third method is designed to direct an ultrasonic beam to the free surface of the liquid, thereby causing the liquid to release minute drops under a local high pressure. It quickly produces minute drops about 1 µm in diameter without requiring any nozzle.

Of the above-mentioned ink jet printing methods, the piezoelectric ink jet method is suitable for the present invention because it is limited in thermal effect on the medium. Moreover, it can control the drop size according to the shape of applied pulses, which leads to accurate analyses. The drop size may be controlled to be small when the radius of curvature of the liquid surface is small, and vice versa. It can also reduce the radius of curvature of the liquid surface by switching the pulse toward negative to pull the drop surface inward.

The micromechanical spotting method is designed to add each minute drop containing the nucleic acid for detection to the detecting surface on the substrate by means of a print head which carries a microspotting pen, capillary, or tweezers.

It is also possible to employ a dropping device which delivers the medium by means of a conductive voice coil which vibrates by mutual action of induced current generated by electromagnetic induction and external magnetic field.

The second step P₂ may be accomplished while the discoid substrate 1 is rotating and the servo mechanism is reading the position of the detecting element(s) 3 on the substrate. Incidentally, the second step P₂ may proceed simultaneously with the third step P₃ so that an electric field is applied to the medium which has been dropped on or injected into the reaction area R. See Step P₂₁ shown in Fig. 13.

(3) The third step P₃ for application of electric field (to immobilize the nucleic acid for detection)

The third step P₃ proceeds in the following manner after or simultaneously with the second step P₂, as shown in Figs. 12 and 13, respectively. This step is intended for application of an electric field to the medium held in the reaction area R on the substrate 1 through the opposing electrodes (say, E₁ and E₂) facing the reaction area R.

The medium in the reaction area R free of electric field contains a DNA probe (which is the single-strand nucleic acid D for detection) in a free state. In its free state, the nucleic acid D takes on a randomly coiled round high-order structure due to Brownian motion.

In the step P₃ (shown in Fig. 12) or the step P₂₁ (shown in Fig. 13), the medium in the reaction area R is given a high-frequency AC electric field by the opposing electrodes (say, E₁ and E₂) connected to an external power source. The high-frequency electric field should preferably have a voltage equal to or higher than 1 MV/m and a frequency equal to or higher than 500 kHz, specifically about 1 × 10⁶ V/m and about 1 MHz. See Masao Washizu and Osamu Kurosawa: "Electrostatic Manipulation of DNA in Microfabricated Structures", IEEE Transaction on Industrial Application vol. 26, No. 26, p. 1165 to 1172 (1990).

The thus applied electric field produces the electrodynamic effect for electrophoresis, which stretches the randomly coiled nucleic acid D for detection and causes it to migrate toward the electrode surface with concentrated electric flux lines. Thus, the nucleic acid D for detection as a DNA probe has its modified terminal immobilized onto the electrode surface through coupling reactions, as shown in Fig. 4. In other words, the electrode surface functions as the detecting surface U. Moreover, the electric field accelerates the migration of the nucleic acid for detection, thereby reducing the processing time.

As mentioned before, the electrode surface treated with streptavidine is suitable for the biotinylated DNA probe to have its terminal immobilized. Likewise, the electrode surface treated with thiol (SH) groups is suitable for the thiol-modified DNA probe to have its terminal immobilized through the disulfide (-S-S-) linkage.

(4) The fourth step P₄ for removal (washing)

The fourth step P₄ is intended to remove and recover an excess of the nucleic acid D for detection (remaining free without being immobilized) from the reaction area R.

This step may be carried out in one of two ways. The first one including dropping or injecting a previously prepared cleaning buffer solution into the reaction area and recovering the cleaning solution together with an excess of the nucleic acid for detection from the reaction area R.

In the foregoing procedure, the cleaning solution can be recovered efficiency by capillary action through a capillary (not shown) communicating with the reaction area R of the substrate 1. In addition, cleaning may be accelerated by centrifugal force resulting from the rotating discoid substrate 1.

Incidentally, the cleaning solution may be a buffer solution of SSC (saline-sodium citrate) containing a surface active agent such as SDS.

The second way of the cleaning step is by application of an electric field through a pair of additional opposing electrodes (say, E₃ and E₄ in Fig. 6) installed separately from the opposing electrodes for immobilization. The electric field applied for cleaning is weak enough not to remove the immobilized nucleic acid for detection but strong enough to move it to the area having no effect on the detecting surface or the vicinity of the surface of the electrodes E₃ and E₄. The thus moved excess nucleic acid is recovered or trapped (See Fig. 7).

The second way mentioned above is not intended to remove an excess of the nucleic acid D for detection from the reaction area R but is intended to move an excess of the nucleic acid D for detection to any area not detrimental to detection in the reaction area R.

Thus, cleaning in the second way is effective in the case where the user performs both the operation of immobilization and the operation of hybridization or in the case where the user performs the operation of hybridization by using the DNA chip 10 which has the nucleic acid D for detection immobilized thereon.

Incidentally, Fig. 7 schematically shows molecules of hybridized double-strand nucleic acid on the surface of the electrode E₁ and also shows excess substances B being attracted to the vicinity of the surface of the electrodes E₃ and E₄.

The substrate 1 processed by the foregoing steps has the nucleic acid D for detection immobilized on the detecting surface U of the reaction area R and an excess of the nucleic acid D for detection removed from the reaction area R. Thus, the substrate 1 is now ready for packaging and shipment to users. It will be referred to as "DNA chip" having integrated gene information. The DNA chip will be indicated by a reference numeral 10 hereinafter. The foregoing is concerned with probe immobilization by application of an electric field. However, an electric field is not necessarily essential because the probe is immobilized by chemical reactions.

### <3. About the system for production of DNA chip and the system for detection of hybridization>

The following covers a typical embodiment of the system for production of DNA chip from the substrate 1 mentioned above and the system for detection of hybridization by the DNA chip pertaining to the present invention. Fig. 14 is a block diagram for the embodiment.

The system shown in Fig. 14 includes means for dropping or injecting the medium, a servo mechanism, and a fluorescence detecting means. The first two are used in both the system for immobilizing the nucleic acid D for detection (or for production of DNA chips) and the system for detecting hybridization. The last one is used also in the system for detection of hybridization.

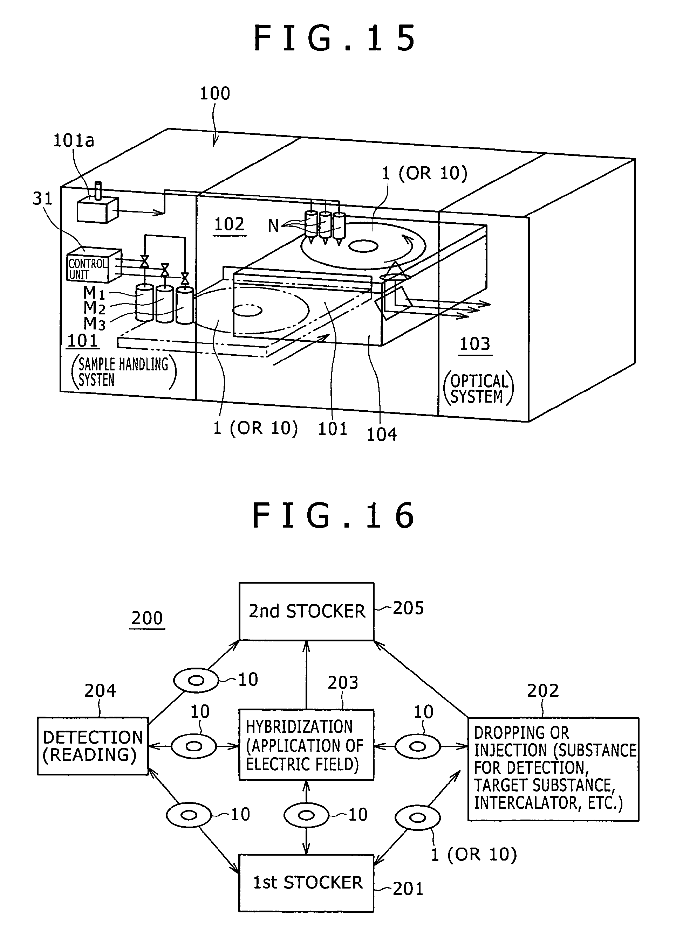

The system shown in Fig. 14 may be divided into two units -- one for production of DNA chips and one for detection of hybridization. Each unit may be designed for specific purposes. This holds true for the apparatus shown in Fig. 15 and the system shown in Fig. 16.

The substrate 1 (or the DNA chip 10) shown in Fig. 14 is constructed as mentioned above. Before operation, it is fixed to the spindle 9 which projects upward from the disc support 8 provided with a rotating means. The spindle 9 penetrates through the central hole 2 (See Fig. 1) of the substrate 1 (or the DNA chip 10).

For production of the DNA chip, the reaction area R in the substrate is given by dropping or injection the medium M₁ containing the nucleic acid D for detection. For detection of hybridization, it is also given by dropping or injection the medium M₂ containing the target nucleic acid T.

The media M₁ and M₂ usually take on the form of liquid or gel with a properly adjusted viscosity. The substrate 1 should be held horizontal while a liquid medium M is being dropped or injected so that it stays in the prescribed place.

The system shown in Fig. 14 has a plurality of nozzle heads N₁ arranged above the upper surface 1c of the substrate 1 (or the DNA chip 10). The nozzle heads N₁ are so constructed as to drop or inject the medium M (say, M₁ to M₃) into each of the reaction area R of the detecting element 3 at proper time intervals as it moves to a prescribed position.

In Fig. 14, there is schematically shown a control unit 31, which controls the dropping or injection from the nozzle heads M₁ in response to "focus information" for the substrate 1 (or the DNA chip 10) and "tracking information" from the substrate 1.

In Fig. 14, there is shown the excitation light Q, which is used for information reading during detection of hybridization. It emerges from the laser diode 12, passes through the collimator lens L₁ (which makes parallel beams), and turns (90° ) at the dichoric mirror 13.

Then, the excitation light Q turns (90°) again at the mirror 14 placed in the path of light, enters the condenser lens L₂ supported by the actuator 15, and impinges upon (the reaction area R) of the detecting element 3 through the lower side 1d of the substrate 1. Incidentally, the reference numeral 16 denotes signals which are sent from the control unit 31 to control the laser diode driver 17.

The excitation light Q is concentrated by the condenser lens L₂ so that it becomes a small several micrometers on the (lower) surface of the substrate 1 (or the DNA chip 10). The thus concentrated excitation light is utilized most effectively when the medium M₁ containing the nucleic acid for detection or the medium M₂ containing the target nucleic acid is dropped or injected onto the substrate 1 (or the DNA chip 10) through the nozzle head N₁ designed for ink jet printing capable of delivering extremely small solution drops of the order of picoliter.

There may be as many nozzle heads N₁ or ink jet printing nozzles as media, or there may be only one nozzle which is used for different kinds of media after cleaning.

The nozzle heads can drop or inject the medium M₃ containing the intercalator I (shown in Fig. 3) onto the reaction area R simultaneously with the dropping or injection of the medium M₂ containing the nucleic acid for detection or at an adequate time after hybridization. They can also drop or inject the cleaning solution at an adequate time after the step of immobilization.

The object of dropping or injecting the medium into desired addresses on the substrate 1 (or the DNA chip 10) is achieved by the servo mechanism using a laser beam V (mentioned later) which reads address mark information previously recorded on the substrate 1 (or the DNA chip 10).

The DNA chip 10 allows for hybridization to form a complementary double-strand nucleic acid from the nucleic acid D for detection which has previously been immobilized onto (the detecting surface of) the reaction area R and the target nucleic acid T (which is dropped or injected later). The dropping or injection of the target nucleic acid T is followed by or accompanied by the dropping or injection of the medium M₃ which contains the intercalator I that enters and combines with the above-mentioned double-strand nucleic acid into the reaction area R through the nozzle head N₁. Incidentally, the intercalator I may be added to the reaction area R before the dropping or injection of the target nucleic acid T if the medium is properly modified.

Upon irradiation with the excitation light Q, the intercalator emits fluorescence indicated by the symbol F. The fluorescence F passes through the lower side 1d of the substrate 1, turns 90° at the mirror 14 placed under the DNA chip 10, passes through the dichroic mirror 13 (placed in the path of light), and finally turns 90° at the dichroic mirror 18.

The fluorescence F passes upward through the condenser lens L₃ and enters the detector 19. The dichroic mirror 18 reflects the fluorescence F but has a characteristic of transmittance for the laser beam Z for servo control (mentioned later).

The fluorescence F has a much weaker intensity than ordinary optical disc RF signals. Therefore, the detector 19 should preferably be any of highly sensitive ones, such as photomultiplier (photocell) and avalanche photodiode (APD).

The fluorescence F detected by the detector 19 is converted into digital signals 32 (with an adequate number of bits) by the AD converter 20. The digital signals 32 are used, for example, to prepare a map in which the intensity of fluorescence corresponds to the addresses on the substrate 1.

The system and apparatus according to the present invention has the servo mechanism explained in the following.

Under the substrate 1 (or the DNA chip 10) is arranged the condenser lens L₂, which is moved in the focus direction (vertical direction) and the tracking direction (circumferential direction) by the actuator 15 of biaxial voice coil type which is used for the optical disc pickup.

Since the substrate 1 (or the DNA chip 10) is held horizontal and the condenser lens L₂ is placed thereunder so that its optical axis is vertical, the excitation light Q and the laser beam Z for focusing and tracking servo control impinges upon the lower side 1d of the substrate 1 (or the DNA chip 10).

The foregoing structure permits the laser beam Z for servo control to reflect from the lower side 1d of the substrate 1 (or the DNA chip 10) without being affected at all by the medium containing the nucleic acid D for detection and the target nucleic acid T which is placed in the detecting element 3 of the substrate 1 (or the DNA chip 10).

In other words, the laser beam Z for servo control reflects while keeping its direction of reflection and its intensity without being disturbed by the medium in the substrate 1 (or the DNA chip 10). This contributes to stable servo control free of focusing and tracking errors.

The servo mechanism has the laser diode 22 under control by the driver 23 placed behind it and the collimator lens L₄ placed in front of it, as shown in Fig. 14.

The collimator lens L₄ makes parallel the laser beam Z for servo control. In front of the collimator lens L₄ is the dichroic mirror 25 which has a characteristic of transmittance for the laser beam Z for servo control.

The system shown in Fig. 14 has the astigmatizer L₅ for astigmatism correction of focusing errors. Knife edge correction, skew correction, etc. may also be applicable.

Tracking errors may be corrected by the differential push-pull method or the three-spot method. Both methods need three spots of light on the disc.

The foregoing object is achieved by placing the diffraction grating 24 between the collimator lens L₄ and the dichroic mirror 25 in the laser optical system for servo control, as shown in Fig. 14. The diffracted light of the zeroth order and the (±) first order passes through the condenser lens L₂ and impinges upon the substrate 1 (or the DNA chip 10).

The reflected laser beam for servo control enters the detector 26 shown in Fig. 14. Incidentally, the driver 23 and the detector 26 are controlled by the controller 31 (shown in Fig. 12).

The excitation light Q, the fluorescence F, and the laser beam Z may differ in wavelength from one another. In this case, the reflection layer 11 on the substrate 1 (shown in Fig. 2) should have a certain transmittance for the fluorescence F and a certain reflectance for the laser beam Z.

In other words, the reflection layer 11 may vary in reflectance and transmittance depending on wavelength or may have frequency characteristics like low-pass filters. The reflection layer 11 would not have improved transmittance and reflectance for the fluorescence F and the laser beam Z, respectively, if it responds to only one wavelength. The transmittance of the florescence F and the reflectance of the laser beam Z can be improved with the wavelength dependence.

### <4. About the apparatus and system for treatment of the substrate>

The above-mentioned constituents are combined into an apparatus for treatment of the substrate. An embodiment of the apparatus is schematically shown in Fig. 15. It is suitable for production of DNA chips and/or detection of hybridization. In addition, it may have its constituents arranged in any different manner than illustrated.

In Fig. 15, the entire apparatus is indicated by the reference numeral 100. It integrally includes all the constituents shown in Fig. 14 which are necessary for production of DNA chip 10 and detection of hybridization.

The apparatus 100 includes means 101 for introducing and discharging the substrate 1 (or the DNA chip 10) into and from it, a storage 102 for the substrate 1 (or the DNA chip 10), means 104 functioning as heating means controlling the environment where the nucleic acid D for detection is immobilized and hybridization takes place and humidification means controlling humidity condition, means N for supplying the nucleic acid for detection or supplying the target nucleic acid to the reaction area where the nucleic acid for detection has been immobilized, and means 103 for reading the state of hybridization.

The apparatus 100 also has means (not shown) for applying an electric field to the reaction area R and means (not shown) for cleaning the nucleic acid D for detection. Incidentally, the apparatus may have separately means for introducing the substrate and means for discharging the substrate, instead of having a single means 101 mentioned above.

The apparatus 100 may additionally have means (101a) for automatically amplifying the target nucleic acid T (cDNA) from mRNA extracted from cells and tissues and introducing it into the supply means N (nozzle head N₁). This additional means automates the process for detection of hybridization.

The apparatus 100 may have the supply means N, the environment controlling means 14 including the heating means, the reading means, the substrate inlet, and the substrate outlet arranged in series. Arrangement in this manner simplifies the construction of the apparatus because the substrate moves straight.

The apparatus 100 performs automatically a series of steps at adequate time intervals after the substrate 1 or the DNA chip 10 has been tightly enclosed in the storage 102, in which the temperature and humidity suitable for assay are established by the environment controlling means 104. The series of steps include dropping or injection of the nucleic acid D for detection, immobilization of the nucleic acid D for detection, dropping or injection of the target nucleic acid T and intercalator, application of an electric field, hybridization, cleaning, or removal, and detection (or reading).

The apparatus 100 is designed to treat the substrate which has thereon the reaction area for hybridization.

That is, the apparatus 100 performs the following steps sequentially and continuously as an additional step for increased hybridization efficiency and improved detection accuracy. The steps include: supplying the target nucleic acid T to the reaction area R in which the nucleic acid D for detection has been immobilized (This step may be accompanied by humidification.); controlling the temperature of the substrate 1 (or the DNA chip 10) which has been supplied with the target nucleic acid T; reading (optically) the state of hybridization in the reaction area R; applying an electric field to the reaction area R of the substrate.

Humidification added to the step of supplying the nucleic acid prevents the medium (or solvent) from drying. Moreover, the temperature control allows for hybridization between the nucleic acid D for detection, which has been immobilized onto the reaction area R, and the target nucleic acid T at an optimum temperature.

The system for treatment of the substrate that can be used for production of the DNA chip and/or detection of hybridization is shown in Fig. 16 which is a schematic block diagram.

The automatic treatment system 200 shown in Fig. 16 is suitable for a large amount of works involving immobilization and detection of hybridization. The concept and construction of the automation system may be applied to the above-mentioned apparatus 100 for treatment of the substrate.

The system 200 is provided with a first stocker 201 which stores a prescribed number of the substrate 1 (without immobilized nucleic acid) or the DNA chip 10 (with immobilized nucleic acid). The first stocker 201 has an air-tight space and means for controlling temperature and humidity.

The first stocker 201 automatically identifies the stored substrate 1 or DNA chip 10 and sends it to another stage. It also identifies the substrate 1 or DNA chip 10 received from another stage and stores it at a designated position.

The system 200 is also provided with the spotting stage 202 which moves the substrate 1 (or the DNA chip 10) back and forth between it and the first stocker 210. The spotting stage 202 contains the nozzle head N₁, the medium storage, and the means for controlling the dropping or injection of the medium (as shown in Fig. 14).

The system 200 is also provided with the hybridization stage 203 which includes means for applying an electric field, means for controlling the electric field, and means for controlling temperature and humidity.

The hybridization stage 203 moves back and forth the substrate 1 (or the DNA chip 10) between the first stocker 201 and the spotting stage 202.

There may be more than one unit of the spotting stage 202. In this case, they receive different kinds of nucleic acid D for detection.

The system 200 shown in Fig. 16 is provided with the detecting (reading) stage 204, which has means for the optical system (shown in Fig. 14) for detection and servo control, means for rotating the DNA chip 10, and a servo mechanism.

The detecting stage 204 is intended to detect hybridization that has occurred in the detecting element 3 of the DNA chip 10. It is connected to an analyzing unit and a display unit (both not shown). It moves the DNA chip 10 to and from the hybridization stage 203 or the first stocker 201.

The system 200 shown in Fig. 16 is provided with a second stocker 205, in which the DNA chip 10 is recovered and stored after the step of detection by the detecting stage 204. The second stocker 205 may be so constructed as to receive and recover the substrate 1 or the DNA chip 10 from the spotting stage 202 or the hybridization stage 203.

As mentioned above, the system 200 may be effectively used to perform assay quickly on a large amount of samples.

### <5. About the method for detection of hybridization>

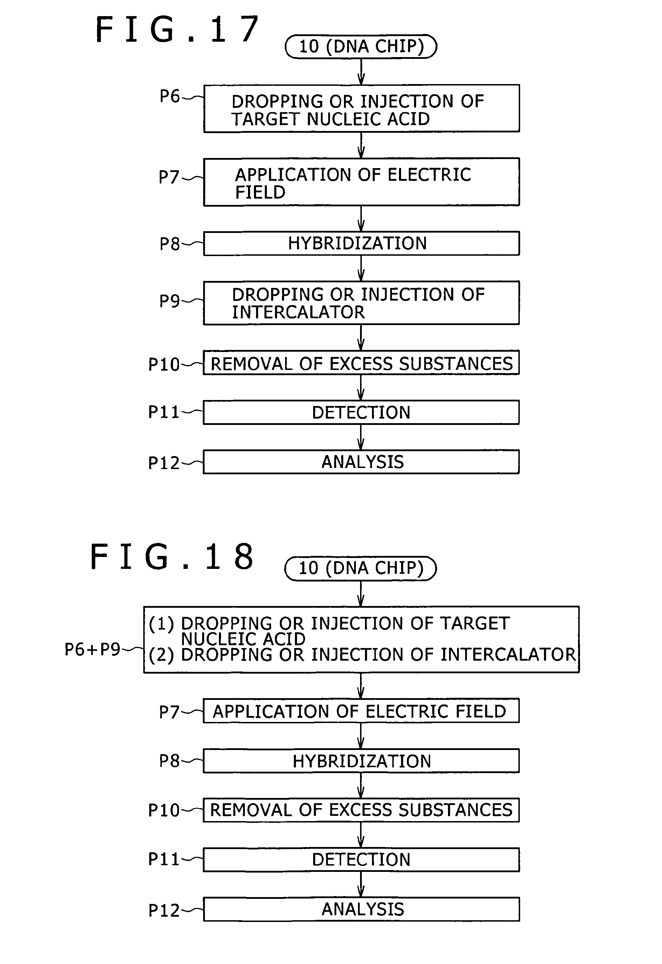

According to the present invention, hybridization is detected by the method which will be described below with reference to Figs. 14, 17, and 18. Fig. 17 is a flow sheet showing one way of detecting hybridization, and Fig. 18 is a flow sheet showing another way of detecting hybridization.

Detection of hybridization starts with mounting the DNA chip 10 horizontally on the disc support 8 (shown in Fig. 14). The DNA chip has the nucleic acid D for detection which as already been immobilized thereon. The DNA chip 10 is turned, with the focusing servo control and tracking servo control working. The detecting element 3 selected according to address information is given the medium M₂ containing the nucleic acid for detection from the nozzle head N₁ by dropping or injection. (This step is indicated by P₆ in Fig. 17.)

The medium held in the reaction area R is given an electric field applied by the opposing electrodes (such as E₁ and E₂ shown in Fig. 4). The electric field produces the effect of electrophoresis which stretches the immobilized nucleic acid (D) for detection and also stretches the target nucleic acid (T) which is present in a free state in the reaction area R and moves it to the detecting surface U. This step is indicated by P₇ in Fig. 17.

Hybridization is allowed to proceed by natural Brownian motion between the nucleic acid D for detection and the target nucleic acid T, with the electric field kept on or turned off. (This step is indicated by P₈ in Fig. 17.)

According to the present invention, hybridization completes in a very short time because the application of an electric field eliminates steric hindrance and other problems. The steps P₇ and P₈ may be carried out separately or simultaneously.

The above-mentioned procedure may be so modified as to accelerate hybridization between the nucleic acid D for detection and the target nucleic acid T by keeping each of the detecting element 3 at an adequate temperature. This object is achieved by providing each of the detecting element 3 with means for heating the medium and means for detecting the temperature of the medium. These means help establish an optimal temperature condition for the reaction between the nucleic acid D for detection and the target nucleic acid T.

In the next step, the reaction area R is given the fluorescence-labeled intercalator I which is dropped or injected from the nozzle head N₁. This intercalator combines with the double-strand nucleic acid which has resulted from hybridization mentioned above (this step is indicated by P₉ in Fig. 17.). This step may be carried out between the step P₇ and the step P₈ as a matter of course. This applies to the flow sheet shown in Fig. 18.

The hybridization area (or the detecting surface area) is subsequently cleared of excess substances B (such as excess target nucleic acid and excess intercalator shown in Fig. 7) and any substance resulting from mishybridization by electrophoresis induced by application of an electric field to the medium held in the reaction area R (this step is indicated by P₁₀ in Fig. 17.).

The step P₉ or P₁₀ is followed by irradiation of the reaction area R with the excitation light Q of specific wavelength and detection of the thus produced fluorescence by the optical pickup means shown in Fig. 14 (This step is indicated by P₁₁ in Fig. 17.).

In this step, the intensity of the fluorescence F emanating from the intercalator is detected to determine the state of hybridization between the nucleic acid for detection and the target nucleic acid. The output from the detector is converted into the digital signals 32 with a specific number of bits by the AD converter 20 (See Fig. 12.).

The foregoing steps make it possible to prepare a map showing correspondence between the address on the DNA chip 10 and the intensity of fluorescence. This map allows for analysis of the target nucleic acid by comparison with the map showing the arrangement of bases immobilized on each reaction area R (this step is indicated by P₁₂ in Fig. 17.).

The above-mentioned procedure may be modified as shown in Fig. 18 such that the step P₆ for dropping or injection of the nucleic acid T for detection and the step P₉ for dropping or injection of the intercalator are carried out at the same time. Combination of two steps P₆ and P₉ improves the efficiency of the procedure.

### <6. About the method for application of an electric field>

The method according to the present invention involves application of an electric field in the following way.

"Application of an electric field" mentioned below may be used in the step of immobilizing the nucleic acid for detection when the DNA chip 10 is produced from the substrate 1 or in the step including its preceding and succeeding steps of hybridization on the DNA chip 10. The application of an electronic field is adaptable in accordance with the effect thereof.

Application of an electric field may be carried out without specific restrictions on field intensity, frequency, and duration of application. These variables should be properly selected according to the kind and length of the nucleic acid. A power source with any waveform including sinusoidal as well as triangular may be used.

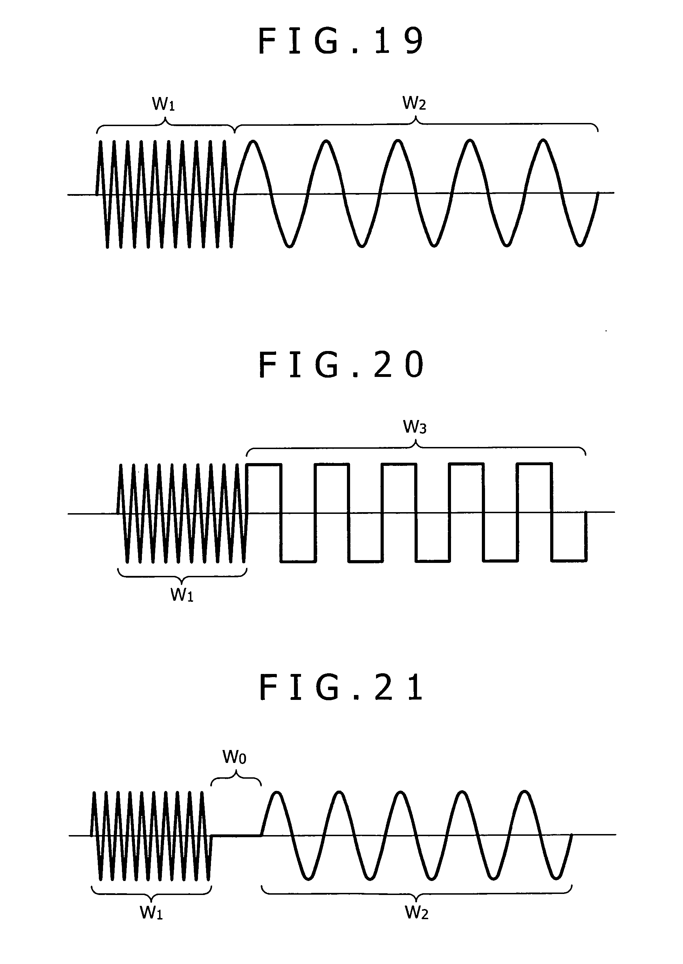

Fig. 19 shows an example of waveforms of electric fields. The symbols W₁ and W₂ denote respectively a high-frequency electric field and a succeeding low-frequency electric field. Fig. 20 shows another example of waveforms of electric fields. The symbols W₁ and W₃ denote respectively a high-frequency electric field and a succeeding low-frequency electric field with a rectangular waveform.

The sequence of application of an electric field shown in Fig. 19 may be modified as shown in Fig. 21. In the modified sequence, a period of null electric field (indicated by W₀ in Fig. 21) is interposed between application of a high-frequency electric field W₁ and application of a low-frequency electric field W₂. The null electric field permits the target nucleic acid which has gathered around the electrode by electrophoresis to undergo hybridization naturally by Brownian motion.

The sequence of application of an electric field shown in Fig. 20 may also be modified by interposing a period of null electric field W₀ as in the case just mentioned above.

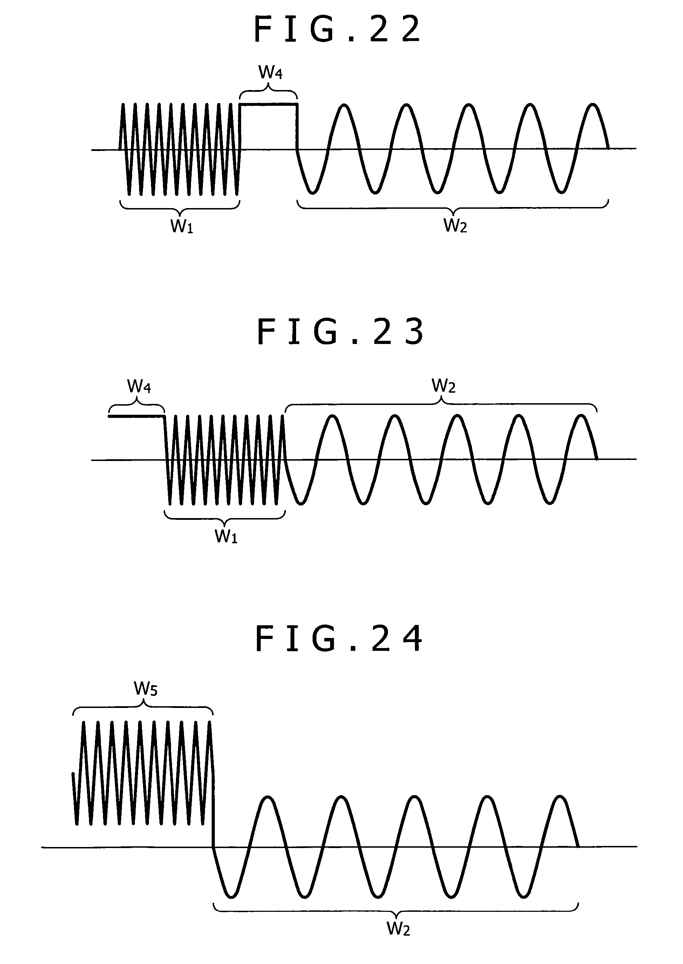

Application of an electric field may also be modified as shown in Fig. 22 by interposing a (DC) electric field W₄ between the high-frequency electric field W₁ and the low-frequency electric field W₂. The interposed DC electric field enhances the effect of attracting the target nucleic acid toward the electrode by electrophoresis.

Application of an electric field may also be modified as shown in Fig. 23 by placing a DC electric field W₄ before the high-frequency electric field W₁ and succeeding the low-frequency electric field W₂. The DC electric field previously attracts the target nucleic acid toward the electrode by electrophoresis.

Application of an electric field may also be modified as shown in Fig. 24 by superimposing a DC component on the high-frequency electric field as indicated by W₅. The superimposed DC component enhances the effect of attracting the target nucleic acid toward the electrode by electrophoresis.

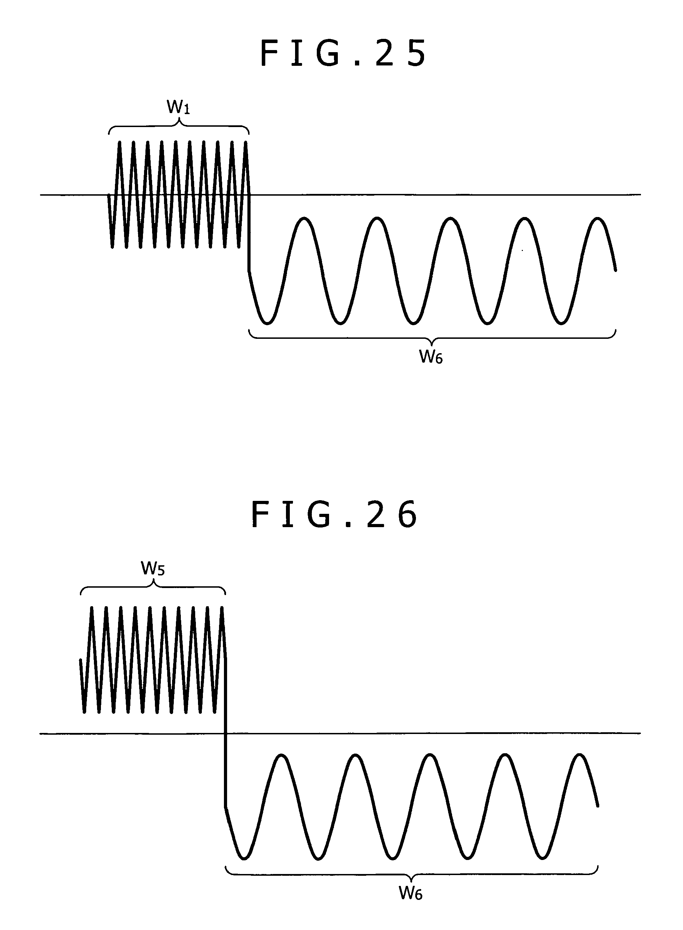

Application of an electric field may also be modified as shown in Fig. 25 by superimposing a DC component on the low-frequency electric field as indicated by W₆. The superimposed DC component enhances the effect of repelling unnecessary nucleic acids from the electrode by electrophoresis. Unnecessary nucleic acids include excess non-complementary target nucleic acid, mishybridized nucleic acids, and complementary target nucleic acid with incomplete hybridization due to steric hindrance.

Application of an electric field may also be modified as shown in Fig. 26 by superimposing a DC component on both the high-frequency electric field as indicated by W₅ and the low-frequency electric field as indicated by W₆. The superimposed DC component enhances the effect of attracting the target nucleic acid toward the electrode by electrophoresis and the effect of repelling unnecessary nucleic acids from the electrode by electrophoresis. Unnecessary nucleic acids include excess non-complementary target nucleic acid, mishybridized nucleic acids, and complementary target nucleic acid with incomplete hybridization due to steric hindrance.

Application of an electric field shown in Figs. 19 to 26 may be modified by replacing W₂ with W₃ (not shown) which is a low-frequency AC electric field. It may also be modified by placing a period of null electric field W₀ before the low-frequency electric field W₂ or the low-frequency AC electric field W₃. This additional period permits the target nucleic acid, which has gathered around the electrode by electrophoresis, to undergo hybridization naturally by Brownian motion.

### [Industrial Applicability]

The method according to the present invention allows for efficient hybridization in a very small reaction area on the substrate, thereby significantly saving time for hybridization. Moreover, it creates a condition and environment suitable for accurate hybridization, thereby suppressing false positive and false negative and greatly improving the accuracy of detection.

The apparatus, system, and method according to the present invention will be used for quick and accurate detection of hybridization.

## Claims

1. A method for production of a DNA chip from a substrate having detecting elements, each provided with at least a reaction area for hybridization and opposing electrodes so arranged as to apply an electric field to a medium held in said reaction area, said method comprising the steps of:
(1) performing surface treatment on said substrate;
(2) applying through said opposing electrodes an electric field to the medium containing a previously prepared nucleic acid probe which is held in said reaction area, thereby immobilizing said nucleic acid probe onto said electrode surface; and
(3) removing an excess of said nucleic acid probe from said reaction area.

2. The method for production of a DNA chip as defined in Claim 1, wherein the step (1) is intended to make said substrate compatible with said medium.

3. The method for production of a DNA chip as defined in Claim 1, wherein said reaction area holds said medium which is dropped or injected thereinto.

4. The method for production of a DNA chip as defined in Claim 1, wherein said substrate is a discoid one.

5. The method for production of a DNA chip as defined in Claim 1, wherein said medium is dropped or injected while said discoid substrate is rotating.

6. The method for production of a DNA chip as defined in Claim 1, wherein the step (2) includes an additional step of moving said nucleic acid probe by electrophoresis induced by application of an electric field.

7. The method for production of a DNA chip as defined in Claim 1, wherein said electric field is a high-frequency one.

8. The method for production of a DNA chip as defined in Claim 7, wherein said high-frequency electric field has a voltage no lower than 1 MV/m and a frequency no lower than 1 MHz.

9. A system for production of a DNA chip from a substrate having detecting elements, each provided with at least a reaction area for hybridization and opposing electrodes so arranged as to apply an electric field to a medium held in said reaction area, said system comprising:
means for dropping or injecting a medium containing a previously prepared nucleic acid probe into said reaction area; and
means for applying an electric field through said opposing electrodes to said medium held in said reaction area.

10. The system for production of a DNA chip as defined in Claim 9, which further comprises means for dropping or injecting said medium into said reaction area and permitting said medium to be held in said reaction area.

11. The system for production of a DNA chip as defined in Claim 9, which further comprises means for removing an excess of said nucleic acid probe from said reaction area.

12. The system for production of a DNA chip as defined in Claim 9, wherein said means for application of an electric field is one which applies a high-frequency electric field.

13. The system for production of a DNA chip as defined in Claim 9, which further comprises means for rotating said substrate.

14. The system for production of a DNA chip as defined in Claim 13, wherein said means for dropping or injection supplies the medium to said reaction area in synchronism with rotation of said substrate.

15. A method for detection of hybridization on a substrate having detecting elements, each provided with at least a reaction area for hybridization, opposing electrodes so arranged as to apply an electric field to a medium held in said reaction area, and a nucleic acid probe which is immobilized in said reaction area, said method comprising the steps of:
(1) dropping or injecting a medium containing a previously prepared target nucleic acid into said reaction area;
(2) applying an electric field through said opposing electrodes to said medium held in said reaction area;
(3) causing hybridization to proceed between said nucleic acid probe and said target nucleic acid;
(4) dropping or injecting an intercalator into said reaction area; and
(5) irradiating said reaction area with excitation light of specific wavelength and detecting the intensity of the resultant fluorescence.

16. The method for detection of hybridization as defined in Claim 15, wherein the steps (1) and (4) are carried out at the same time.

17. The method for detection of hybridization as defined in Claim 15, wherein the step (3) is carried out in the presence of electric field.

18. The method for detection of hybridization as defined in Claim 15, wherein the step (3) is carried out in the absence of electric field.

19. The method for detection of hybridization as defined in Claim 15, wherein the step (5) is preceded by a step of removing an excess of the target nucleic acid existing in said reaction area from the hybridization area.

20. The method for detection of hybridization as defined in Claim 19, wherein removal of excess target nucleic acid is accomplished by application of an electric field which attracts excess target nucleic acid to the surface of the electrode outside the hybridization area.

21. The method for detection of hybridization as defined in Claim 15, wherein said substrate has a layer that transmits said excitation light.

22. The method for detection of hybridization as defined in Claim 15, wherein said excitation light is directed to the lower side of said substrate.

23. The method for detection of hybridization as defined in Claim 15, wherein the step (2) for application of an electric field is performed on said substrate all at once or on each block of areas.

24. The method for detection of hybridization as defined in Claim 15, wherein the step (1) and/or the step (4) are carried out while said substrate is rotating.

25. The method for detection of hybridization as defined in Claim 23, wherein the step (5) is carried out while said substrate is rotating.

26. A system for detection of hybridization on a substrate having detecting elements, each provided with at least a reaction area for hybridization, opposing electrodes so arranged as to apply an electric field to a medium held in said reaction area, and a nucleic acid probe which is immobilized in said reaction area, said system comprising:
(1) means for dropping or injecting a medium containing a previously prepared target nucleic acid into said reaction area;
(2) means for applying an electric field through said opposing electrodes to said medium held in said reaction area;
(3) means for causing hybridization to proceed between said nucleic acid probe and said target nucleic acid;
(4) means for dropping or injecting an intercalator into said reaction area; and
(5) means for irradiating said reaction area with excitation light of specific wavelength and detecting the intensity of the resultant fluorescence.

27. The system for detection of hybridization as defined in Claim 26, wherein said substrate has a layer that transmits said excitation light.

28. The system for detection of hybridization as defined in Claim 26, wherein said excitation light is emitted from a source facing the reverse side of said substrate.

29. The system for detection of hybridization as defined in Claim 26, which further comprises means for controlling temperature and/or means for controlling humidity for said hybridization.

30. The system for detection of hybridization as defined in Claim 26, which further comprises means for supplying water to said reaction area.

31. The system for detection of hybridization as defined in Claim 26, which further comprises means for rotating said substrate.

32. The system for detection of hybridization as defined in Claim 26, which further comprises a servo control means for positioning said detecting element on said substrate and a servo control means for focusing.

33. An apparatus for treating a substrate having thereon reaction areas for hybridization, said apparatus comprising:
(1) means for supplying a target nucleic acid to said reaction areas where a nucleic acid probe has been immobilized;
(2) means for heating to a controlled temperature said substrate which has been supplied with said target nucleic acid;
(3) means for reading the state of hybridization on said substrate; and
(4) means for transporting said substrate.

34. The apparatus for treating a substrate as defined in Claim 33, which further comprises means for applying an electric field to said substrate.

35. The apparatus for treating a substrate as defined in Claim 33, wherein said means for reading is one for optical reading.

36. The apparatus for treating a substrate as defined in Claim 33, wherein said means for transportation continuously transports said substrate through said means for supplying, said means for heating, and said means for reading.

37. The apparatus for treating a substrate as defined in Claim 33, wherein said means for supplying, said means for heating, and said means for reading are arranged in series.

38. The apparatus for treating a substrate as defined in Claim 33, wherein said means for supplying has means for humidifying.

39. The apparatus for treating a substrate as defined in Claim 33, which further comprises:
means for introducing said substrate to said means for supplying; and
means for discharging said substrate from said means for reading.

40. The apparatus for treating a substrate as defined in Claim 39, wherein said means for introducing said substrate and said means for discharging said substrate are combined into one unit.

41. The apparatus for treating a substrate as defined in Claim 39, wherein said means for supplying, said means for heating, said means for reading, the inlet for said substrate, and the outlet for said substrate are arranged in series.

42. The apparatus for treating a substrate as defined in Claim 33, which further comprises a second means for supplying the solvent to said reaction area in which the nucleic acid probe is immobilized.

43. The apparatus for treating a substrate as defined in Claim 33, which further comprises means for extracting nucleic acids from blood.

44. A method for treating a substrate having thereon reaction areas for hybridization, said method comprising the steps of:
supplying a target nucleic acid to said reaction areas where a nucleic acid probe has been immobilized;
heating to a controlled temperature said substrate which has been supplied with said target nucleic acid; and
reading the state of hybridization in said reaction area;
with said steps being performed consecutively.

45. The method for treating a substrate as defined in Claim 44, which further comprises a step of applying an electric field to said substrate.

46. The method for treating a substrate as defined in Claim 44, wherein said step for temperature control gives a period in which hybridization takes place between said nucleic acid probe and said target nucleic acid.

47. The method for treating a substrate as defined in Claim 44, wherein said step for reading is one for optical reading.

48. The method for treating a substrate as defined in Claim 44, wherein said substrate is transported through the step of supplying said nucleic acid, the step of controlling temperature, and the step of reading.

49. The method for treating a substrate as defined in Claim 44, wherein the step of supplying said nucleic acid involves humidification.
